# EUROPEAN PATENT SPECIFICATION

(11) **EP 4 087 528 B1**
(45) Date of publication and mention of the grant of the patent: **06.08.2025**
(21) Application number: 20845703.6
(22) Date of filing: 31.12.2020
(51) Int. Cl.: A61H 35/04, A61M 3/02, B05B 1/02, A61H 33/02, A61M 15/08, B05B 1/34, B65D 83/28

(54) **A LIQUID DISPENSER**
SPENDER
DISTRIBUTEUR

(30) Priority: 08.01.2020 IT 202000000163
(43) Date of publication of application: 16.11.2022
(73) Proprietor: Mezzoli, Giorgio, 48022 Lugo (RA) (IT); Rani, Maria, 48022 Lugo (RA) (IT)
(72) Inventor: Mezzoli, Giorgio, 48022 Lugo (RA) (IT); Rani, Maria, 48022 Lugo (RA) (IT)
(74) Representative: Roncuzzi, Davide
(86) International application number: PCT/IB2020/062579
(87) International publication number: WO 2021/140412

(56) References cited:
- WO-A1-01/28607
- WO-A1-96/29044

## Description

The present invention concerns a liquid dispenser. In particular, the present invention concerns a liquid dispenser comprising a membrane body provided with at least one nozzle in apical position. In further detail, the present invention concerns a liquid dispenser comprising a membrane body provided with at least one nozzle in apical position to effectively spray human or animal body cavities.

### DESCRIPTION OF THE STATE OF THE ART

The nasal cavities form the uppermost part of the respiratory tract and are separated by the nasal septum, which is an osteo-cartilaginous wall coated by mucosa. Each cavity is split into areas having different anatomical shapes according to their natural functions. A summary description of these areas is provided below.

The area of the nasal vestibule is the outermost part of the nose. The nasal vestibule is medially delimited by a rigid component, the nasal septum, and delimited laterally and at the top by an elastic component, the wings of the nose. The structures that compose the nasal vestibule give it the shape of an oval or elliptical funnel, the coronal section area of which tapers from the outside towards the inside. The nasal vestibule has the function of conveying the air inhaled towards the nasal cavity.

The valve area: the nasal valve or ostium internum is the transition point between the nasal vestibule and the nasal cavity. The upper part of the valve area is called nasal valve, and consists of the junction between the rigid component, namely the nasal septum, arranged medially, and the elastic component of the wing of the nose, arranged supero-laterally. It is the elastic structure that regulates the direction and flow rate of the air currents, during both inhalation and exhalation.

The area of the turbinates "nasal cavity": each nasal cavity communicates at the front with the nasal vestibule through the nasal valve and at the rear with the nasopharynx through the choanal area; medially it is delimited by the nasal septum and laterally by the lateral wall from which bony convolutions called nasal turbinates, which are covered by a highly vascularized and innervated mucosa. There are generally 3 nasal turbinates in each nasal cavity - lower, middle and upper; they are positioned between the nasal septum and the lateral wall of the nasal cavity and delimit irregular spaces called lower meatus, middle meatus and upper meatus respectively. In the upper and middle part of the nasal cavity, below the cribriform plate, the air space of which is reduced to a width of 1-2 mm, is the olfactory region, located above the middle nasal turbinate, between the nasal septum and the lateral wall. The olfactory region is covered by olfactory epithelium, present only in this region. Each nasal cavity communicates, through small ducts, with the respective paranasal sinuses: the maxillary sinuses, the frontal sinuses and the anterior ethmoid sinuses are connected to the middle meatus; the rear ethmoid sinuses and the sphenoid sinuses open into the upper meatus.

The choanal area is the terminal part of the nasal cavity through which each nasal cavity communicates with the nasopharyngeal area. The nasopharyngeal area is very important since it communicates with the cavity of the middle ear through the Eustachian tube and due to the presence of the adenoid tissue.

The nasal mucosa consists of a pseudo-stratified epithelium in which the majority of the cells are ciliated cells, caliciform cells and basal cells; the epithelium rests on a basal membrane and the latter on the plate in which capillaries, arterio-venous anastomoses, seromucous glands and cavernous sinusoids are present. The cilia are immersed in a "sol" aqueous fluid, produced by the anterior serous glands, by the seromucous glands, by the transudate of the vessels and by the condensed water of the exhaled air; on the surface there is a very thin layer of sticky mucus, a sort of "gel" produced by the caliciform cells and by the seromucous glands. The pH of the nasal mucosa is approximately 5.5-6.5. The cilia move rhythmically at a frequency of 16/sec. i.e., 1000/min., determining the movement of the mucous surface layer towards the oropharynx which, in normal conditions, takes place in approximately 12 min. (the transport speed is extremely variable from 3 to 25 mm/min.); in children the mucous is carried towards the adenoids, increasing contact with the immune system. The mucociliary system serves to humidify, warm and cleanse the inhaled air. The body temperature is ideal for the ciliary movement. The temperature influences the mucociliary system: between 32 and 40°C the ciliary movement reaches maximum functionality and remains constant. The mucociliary system constitutes a natural barrier which has the function of protecting against pathogens and cleansing the inhaled substances.

In normal conditions the nasal cavities perform the function of conditioning the inhaled air, warming it, humidifying it and cleansing it, and the exhaled air, reabsorbing heat and humidity. The nasal turbinates make these functions possible by giving the respiratory air a turbulent motion so as to increase the surface inside the nasal cavity in contact with the air.

There are many dispensers of medicated or pharmacologically active solutions on the market that can be used for washing with said solutions the inside of body cavities of humans and animals, including the nasal cavities, the oral cavity, the vaginal cavity and the external ear canal, without minimizing the multiplicity of use of said devices. A large part of said dispensers have been conceived and designed for washing the nasal cavities. Washing of the nasal cavities is performed by causing the washing solution to flow inside a nasal cavity passing through the nasopharynx and allowing the solution to flow out through the other nasal cavity. In this way any pathological secretions present are mechanically removed. Throughout the nasal washing the user must keep his/her head tilted forward and breath through the mouth which must remain open. This prevents some of the solution getting into the lower airways causing coughing and, in the most serious cases, bronchospasm. It is evident that these devices are not suitable for carrying out nasal washes on patients of all ages, in particular in babies or non-collaborative patients who tend not to tolerate nasal washes. In these cases, it is preferable to administer the solutions, also medicated, in the form of spray, aerosol or micronized. Of the known devices, the most effective one is the device produced by the applicant, applying the teachings contained in its own Italian patent IT 1318646, the teachings of which are considered incorporated in the present description for the sake of practicality. Said dispenser has a domed body constructed symmetrically with respect to two median planes that cross in a central axis and, at the apex, one or two openings respectively circular or semi-circular or elliptical through which a circular jet or two semi-circular or elliptical jets flow out respectively and are oriented directly onto the walls of the nasal cavity; in view of the extreme delicacy of the muco-ciliary system present on the surface of the nasal mucosa, said jets can damage the protective barrier formed by the muco-ciliary system and favour the penetration of pathogens. The search for alternative solutions that can avoid the negative effects described has induced the applicant to consider even more advanced embodiments of dispensers which on the one hand allow distribution of the washing liquid to be further increased and, on the other, provide an increase in the degree of turbulence of the liquid inside the anatomical cavities to be treated, which may be not only nasal, but oral, the external ear canals or the vaginal cavity, to define a new standard of dispensers optimized with medical solutions that limit and overcome the drawbacks typical of the known state of the art illustrated above. In order for washing of the nasal cavities to be effective, it is necessary for the washing liquid to be distributed inside the nasal cavity so as to reach the mucosa that covers the anatomical structures present, guaranteeing that the washing liquid is administered and diffused in such a way that the jet or the jets, carried into the nasal cavity, on the one hand do not alter the protective barrier formed by the mucociliary system present on the surface of the mucosa inside the nasal cavity and, on the other, are able to mechanically remove even dense mucus, dried mucus and any pathogens present.

Examples of liquid dispensers according to the known art to spray nasal cavities are known from the documents WO 96/29044 and WO 01/28607.

Document WO9629044A1 discloses a liquid dispenser for medical uses wherein a first membrane body is shaped symmetrically with respect to a first front median plane and to a second sagittal median plane intersected in a central axis and is delimited at the top by a dome; with a central nozzle and at least a first lateral nozzle.

### SUMMARY OF THE PRESENT INVENTION

The present invention concerns a liquid dispenser according to claim 1; further embodiments of the liquid dispenser according to the present invention are defined by at least one subsequent dependent claim. In particular according to one embodiment, the liquid dispenser comprises a membrane body provided with at least one nozzle in apical position. In further detail, according to one embodiment, a liquid dispenser comprises a membrane body provided with at least one nozzle in apical position to effectively spray human or animal body cavities.

The object of the present invention is to provide a dispenser that is without the drawbacks described above and which therefore allows liquid solutions to be dispensed in the form of a jet of substantially nebulized liquid particles uniformly directed towards the described meatus (lower, middle and upper) and the entire mucosa that internally covers each nasal cavity in order to ensure complete spraying thereof.

The problems described above are solved by the present invention according to at least one of the claims.

According to some embodiments a liquid dispenser for medical uses is provided in which a first membrane body is shaped symmetrically with respect to a first frontal median plane and a second sagittal median plane intersected in a central axis and is delimited at the top by a dome; a second body being carried by said first body inside said dome in a position concentric to said axis; said second body having a lower hollow portion concentric with said axis and an upper portion which has a central duct and at least one first lateral duct terminating in said dome, respectively, with a flared central nozzle and at least a first lateral nozzle; said central nozzle having a lateral portion that extends laterally to said second sagittal median plane overlapping each said first lateral nozzle.

According to one embodiment, said central nozzle has a lateral portion which extends laterally to said second sagittal median plane totally overlapping each said first lateral nozzle.

According to one embodiment, a second lateral duct is obtained in said upper portion, where said second lateral duct ends in said dome in a polar symmetrical position relative to said central axis with a second lateral nozzle overlapping with said central nozzle.

According to one embodiment, said central nozzle is flared and has a first lateral portion and a second lateral portion arranged symmetrically relative to said second sagittal median plane, each of which overlapping at least partially one of said first lateral nozzle or second lateral nozzle.

According to one embodiment, each of said first lateral portion and second lateral portion totally overlaps one of said first lateral nozzle or second lateral nozzle.

According to one embodiment, said central duct has a conical shape with section decreasing towards said dome.

According to one embodiment, said first and second lateral ducts are developed on parallel planes.

According to one embodiment, said central nozzle has a section transversal to said axis having oval or rectangular shape.

According to one embodiment, said dome has externally at least one discharge groove.

According to one embodiment, a liquid dispenser for medical use is provided, in which a first membrane body is shaped symmetrically relative to a first frontal median plane and to a second sagittal median plane intersected in a central axis and is delimited at the top by a dome; a second body being carried by said first body inside said dome in a position concentric to said axis; said second body having a lower hollow portion concentric with said axis and an upper portion having a central duct and at least one first lateral duct ending in said dome, respectively, with a central nozzle and at least one first lateral nozzle; where the projection of said central nozzle on a plane perpendicular to said axis has an oval or rectangular shape, where said central nozzle has a first lateral portion, the projection of which on said plane perpendicular to said axis extends laterally relative to said second sagittal median plane, and where the projection on said plane perpendicular to said axis of said at least one first lateral nozzle is positioned entirely laterally relative to said second sagittal median plane.

According to one embodiment, the projection on said first frontal median plane of said at least one first lateral duct at least partially overlaps the projection on said first frontal median plane of said central duct.

According to one embodiment, the projection on said plane perpendicular to the axis of said at least one first lateral nozzle has an oval or rectangular shape.

According to one embodiment, said central nozzle has a second lateral portion, the projection of which on said plane perpendicular to said axis extends laterally with respect to said second sagittal median plane and symmetrically with respect to said projection on said plane perpendicular to said axis of said first portion of central nozzle.

According to one embodiment, said dispenser comprises a second lateral duct ending in said dome with a second lateral nozzle, the projection of which on said plane perpendicular to said axis is positioned entirely laterally relative to said second sagittal median plane.

According to one embodiment, the projection on said first frontal median plane of said at least one second lateral duct at least partially overlaps the projection on said first frontal median plane of said central duct.

According to one embodiment, the projection on said plane perpendicular to the axis of said at least one second lateral nozzle has an oval or rectangular shape.

According to one embodiment, said nozzle is flared and has a section increasing towards said dome.

According to one embodiment, said first and second lateral ducts are developed on parallel planes.

According to one embodiment, said dome has externally at least one discharge groove.

### BRIEF DESCRIPTION OF THE FIGURES

The invention will be better described with reference to some non-limiting embodiments in the attached figures, in which:
- figure 1 is a schematic perspective view from above of a first preferred embodiment of a dispenser;
- figure 2 is a schematic perspective view from below of figure 1 on an enlarged scale;
- figure 3 is a plan view of figure 1 on an enlarged scale;
- figure 4 is a view from below of figure 1 on an enlarged scale;
- figure 5 is a sectional view from below of figure 3 according to a second sagittal plane;
- figure 6 is a sectional view from below of figure 3 according to a first plane advanced and parallel to the sagittal plane;
- figure 7 is a sectional view from below of figure 3 according to a second plane set back from and parallel to the sagittal plane;
- figure 8 is a sectional view from below of figure 3 according to a median plane;
- figure 9 is a sectional view from above of figure 3 according to the median plane of figure 8;
- figure 10 is a sectional view from above of figure 3 according to a third plane set back from and parallel to the median plane of figure 8;
- figure 11 is a sectional view from above of figure 3 according to a fourth advanced plane parallel to the median plane of figure 8;
- figure 12 is a plan view of a second preferred embodiment;
- figure 13 is a view from below of figure 12;
- figures 14-16 are sectional views according to three different planes parallel to the sagittal plane;
- figure 17 is a sectional view from above according to a median plane of figure 12;
- figure 18 is a plan view of a third preferred embodiment;
- figure 19 is a view from below of figure 18;
- figures 20-23 are sectional views according to four different planes parallel to the sagittal plane; and
- figure 24 is a sectional view from above according to a median plane of figure 18.

### DETAILED DISCLOSURE OF THE PRESENT INVENTION

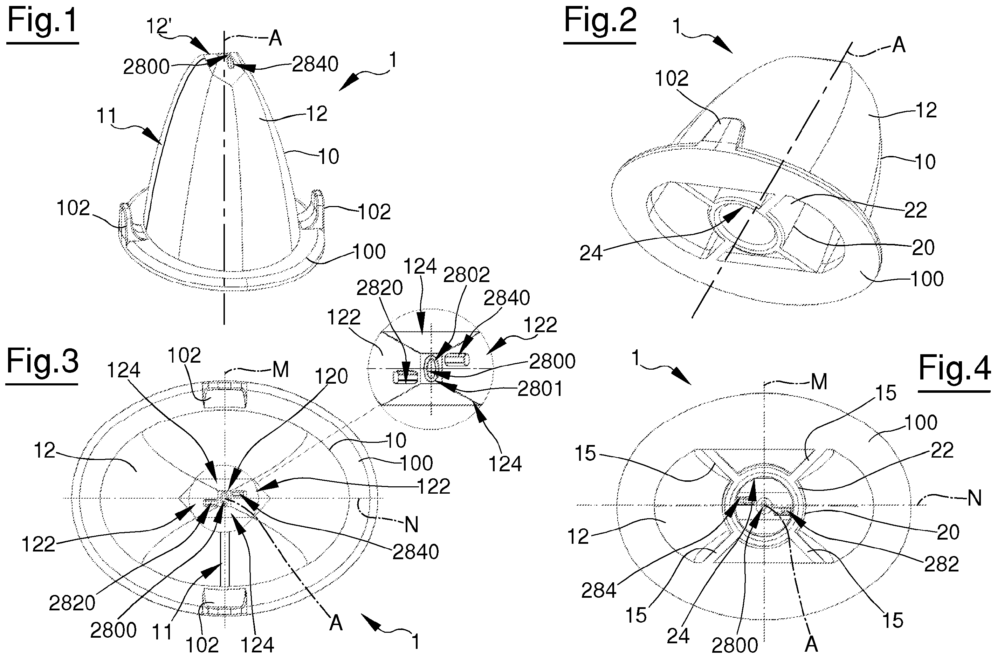

In figure 1, the reference number 1 indicates a liquid dispenser 1 for medical uses supplied under pressure to the same dispenser 1 manually or in any other way. The dispenser 1 comprises a first membrane body 10 shaped symmetrically with respect to a first frontal median plane M and to a second sagittal median plane N intersected in a central axis A, vertical in figure 1. Each of these median planes M and N corresponds to an axis of symmetry of given extension for the sections transversal to the central axis of the first body 10. The oval or elliptical dome shape of the first body 10, where the extension of the first body 10 according to the second sagittal median plane N is greater than the extension of the first body 10 according to the first frontal median plane M, having the same shape as the nasal vestibule and valve, on the one hand eases the insertion thereof inside the nasal vestibule and valve in which the outer wall of the first body 10 adheres to the inner wall of the nasal vestibule and valve and, on the other, functions as a guide to ensure that the jet of the dispenser 1 is always directed in the same way inside the nasal cavity, therefore towards the three meatus and the air space between the wall of the nasal septum and the relative nasal turbinates. The first body 10 is delimited at the bottom by an annular flat body which here and below will be indicated by the term crown 100. The latter is provided with a pair of opposing protrusions 102 oriented substantially parallel to the second sagittal median plane N. Furthermore, both the dispenser 1 and the first membrane body 10 can be made of pressure deformable plastic material such as, for example, PVC, silicone or any other material having the same mechanical characteristics, or also a rigid plastic material such as, for example, but not limited to, ABS without limiting the scope of the present invention. With particular reference to figures 1-3, the first body 10 comprises a substantially dome-shaped portion 12, thus named due to the shape of the respective outer surface, and therefore below referred to simply as dome 12.

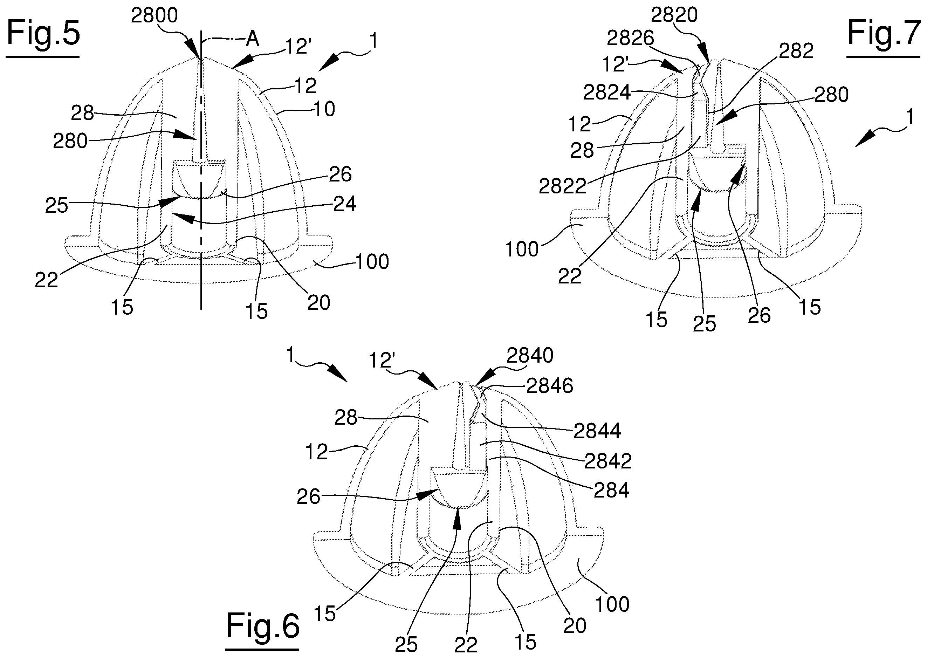

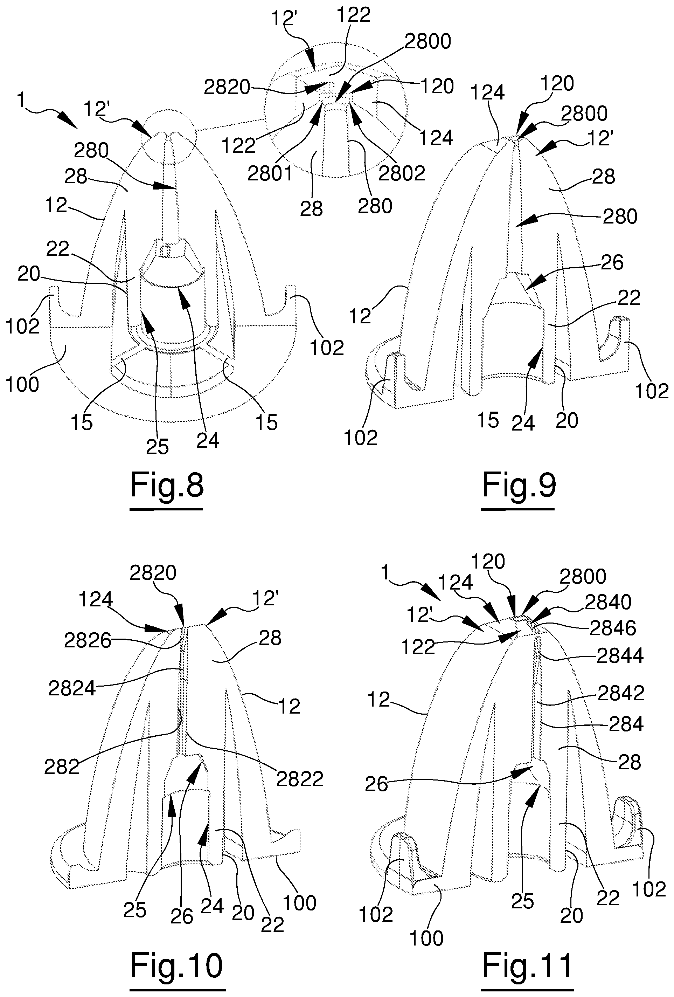

The dispenser 1 comprises a second body 20 which is carried by the first body 10 inside the dome 12 in a position concentric to the axis A through substantially radial ribs 15. The second body 20 has a lower hollow portion 22 (figure 2) concentric with the axis A inside which a cylindrical seat 24 is obtained at the bottom, ending at the top in a tapered portion 26 which can be seen better in figures 8 and 9. The second body 20 furthermore has an upper portion 28 which houses (inside which are obtained) some ducts which are in aerial communication with the tapered portion 26 and establish aerial communication with the outside of the dome 12 through nozzles which will be better described below. In particular, the upper portion 28 has a conical central duct 280 coaxial with the axis A (figures 8 and 9) and two lateral ducts 282 and 284 (figures 6, 7, 10, 11) substantially identical and positioned according to a polar symmetry around the axis A, therefore on opposite sides with respect to an axis of the cross sections of the dome 12 which lies on the second sagittal median plane N. In further detail, the lateral duct 282 and the lateral duct 284 are arranged between the first frontal median plane M and the second sagittal median plane N on opposite quadrants, as can be easily seen in figures 3 and 4, exploiting a geometric analogy with the quadrants detached from an X axis and a Y axis in a Cartesian plane, in which the two mentioned axes are the lines in the plane transversal to the axis A of the median plane M and sagittal plane N of figures 3 and 4, in particular the one associated with the second sagittal median plane N being greater and the one associated with the first frontal median plane M being smaller. The seat 24 and the tapered portion 26 are connected by a step 25, the purpose of which, in use, is to define an abutment for a tube known and not illustrated which can be used to supply liquid to the upper portion 28, and in particular to the central duct 280 and to the two lateral ducts 282 and 284. The central duct 280 ends in a central nozzle 2800 geometrically symmetrical with respect to the second sagittal median plane N. The plan shape of the central nozzle 2800 is an elongated oval and is the same for each section executed with a plane transversal to the axis A of the portion of the central duct 280 leading to the same central nozzle 2800. The largest dimension of each section transversal to the axis A lies on the first frontal median plane M and the two lateral ducts 282 and 284 have rectangular sections preferably with rounded edges throughout the length but said sections can have different geometrical shapes, for example oval, circular, elongated polygonal, circular flattened along a diameter etc., without conditioning the scope of the present invention. In the same way, also the first lateral nozzle 2820 and the second lateral nozzle 2840 are shaped in a rectangular section preferably with rounded edges but they can also be shaped in a functionally equivalent manner, for example, but not limited to, oval, circular, elongated polygonal, circular flattened along a diameter etc., without modifying the functionality of the dispenser or conditioning the scope of the present invention.

With particular reference to figure 3, the dome 12 has a faceted top 12' provided with a central flat portion 120 transversal to the axis A and four flat faces inclined with respect to the axis A, of which two equal opposite first faces 122, symmetrical with respect to the first median plane M, and two equal opposite second faces 124, symmetrical with respect to the second sagittal median plane N. The two lateral ducts 282 and 284 open at the top of the dome, respectively, with a first lateral nozzle 2820 and a second lateral nozzle 2840, both rectangular with rounded vertexes and, further to the above description, inclined with respect to the axis A. Also the first lateral nozzle 2820 and second lateral nozzle 2840 are positioned according to a polar symmetry around the axis A on opposite sides with respect to the second sagittal median plane N. With particular reference to figures 6 and 7 the two lateral ducts 282 and 284 are geometrically identical and develop respectively along planes parallel and equally spaced from the sagittal plane N. Each lateral duct 282/284 has a first part 2822/2842 with constant section in aerial communication with the tapered portion 26; said first part 2822/2842 evolves into a narrowing 2824/2844, the longitudinal section of which is shaped substantially in the form of a rectangular trapezium, continuing with an inclined part 2826/2846 towards the axis A with cross section slightly flared or substantially constant. With reference to the enlargement of figure 8, the shape of the central nozzle 2800 is elongated oval and arranged symmetrically with respect to the second sagittal median plane N, similar to a flattened funnel; in particular, the central nozzle 2800 is flared with dimension increasing towards the outlet and has a first left-hand lateral portion 2801 which is spatially arranged on the same side as the first lateral nozzle 2820. For the sake of practicality, here and below it is appropriate to use the term "overlapping" of the first left-hand lateral portion 2801 of the central nozzle 2800, since the respective projections on a plane parallel to the first frontal median plane M are at least partially overlapped. Further to the above description, and given the symmetry of the dome 12 with respect to the second sagittal median plane N, the central nozzle 2800 has a second right-hand lateral portion 2802 which is spatially arranged on the same side as the second lateral nozzle 2840, according to the known rules of symmetry described above which eliminate the need for an additional figure without compromising understanding of the particular shape of the dispenser 1 overall.

The central nozzle 2800 can have not only an oval shape but, while maintaining its flared configuration, can also be rectangular with rounded edges, circular, elongated polygonal, circular flattened along a diameter etc., without modifying the functionality of the dispenser or conditioning the scope of the present invention.

Further to the above description, the central nozzle 2800 is, in use, supplied with pressurised liquid, the speed of which increases until it reaches the smaller section, located at the base of the nozzle 2800, where the pressure reaches its minimum in accordance with Bernoulli's equation. At this point, the liquid reaches the flaring of the nozzle 2800 where the speed precipitates and the pressure increases causing expansion of the liquid that forms a fan-shaped jet. Said jet expands both in the direction of the first frontal median plane M and in the direction of the second sagittal median plane N in direct proportion to the widths of the nozzle 2800 measured along the directions identified by said planes (greater in the direction of the plane M and smaller in the direction of the plane N). The same thing happens to the liquid supplied to the first and second nozzles 2820 and 2840 through the lateral ducts 282 and 284, for the sequence of the respective first part 2822/2842 with constant section, narrowing 2824/2844 with decreasing section and part 2826/2846 inclined towards the axis A with slightly flared or substantially constant cross section. Therefore the liquid flowing out of the first and second nozzles 2820 and 2840 expands above the top 12' of the dome 12 in substantially nebulized particles.

Further to the above description, the first and second nozzles 2820 and 2840 are shaped to direct at maximum the respective lateral jets against the respective lateral portions of the jet emitted from the central nozzle 2800 which correspond, respectively, to the first left-hand lateral portion 2801 of the central nozzle 2800 and the second right-hand lateral portion 2802. For this reason, each of the jets delivered by the first and second nozzles 2820 and 2840 strikes the corresponding lateral portion of the fan-shaped jet dispensed by the central nozzle 2800, producing a single jet which uniformly expands radially in all directions, reaching all the areas of the mucosa covering the nasal cavity, it would not be possible to reach said areas if the dispenser 1 were provided only with the central nozzle 2800, able to dispense a single jet, albeit fan-shaped, or only with the first and second lateral nozzles 2820 and 2840. Further to the above description, the jet resulting from the combination of the jets delivered under pressure by the central nozzle 2800 and by the first and second nozzles 2820 and 2840 will be indicated here and below by the expression "modified fan-shaped jet".

Furthermore, with particular reference to figures 1 and 3, the dome 12 has externally at least one groove 11, the function of which is to discharge air or liquid supplied in excess to the nasal cavity so that the substantially nebulized liquid can spread as deeply as possible into the nasal cavity and prevent any pressure increase during administration of the liquid nebulized or in the form of spray or aerosol. In figures 1 and 3 only one groove 11 has been shown for the sake of practicality, without conditioning the scope of the present invention.

The use of the dispenser 1 can be easily understood from the above description and does not require further explanation. However, it may be useful to specify that the dispenser 1 is designed to be supplied with liquid under pressure. Furthermore, the first body 10 is shaped for easy treatment of the nasal cavities; the dome 12 is shaped to be pushed at least partially inside a nostril to be treated; once this pushing action has been applied, the outer wall of the dome 12 adheres to the inner walls of the nostril, widening them so that the central nozzle 2800 and the first and second lateral nozzles 2820 and 2840 (of the two lateral ducts 282 and 284) are univocally positioned at the inlet of the nasal cavity facing the 3 meatus.

With the dome 12 of the dispenser 1 kept still in said position, the dispenser 1 has the central nozzle 2800 and the first and second nozzles 2820 and 2840 in an appropriate position for spraying the lower, middle and upper meatus of the nasal cavities through the nozzle 2800 of the central duct 280 and the first and second lateral nozzles 2820 and 2840 with the medical liquid in the form of the "modified open fan-shaped jet".

Lastly it is clear that variations can be made to the dispenser 1 described and illustrated above without departing from the scope of the present invention so that it can be used to administer solutions inside the vaginal cavity, oral cavity and outer ear canal.

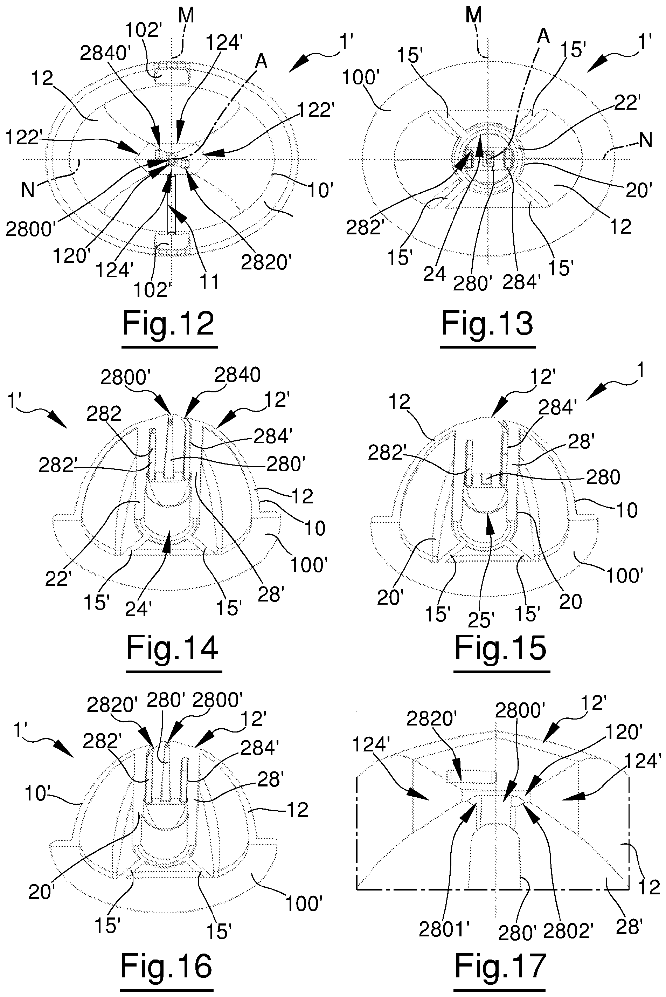

For example, with reference to figures 12-17 a variation of the dispenser 1 indicated by the reference number 1' is illustrated, where homologous structural details will be indicated for the sake of practicality by the same reference numbers as those used in figures 1-11, but provided with an apex.

The dispenser 1' is functionally and aesthetically similar to the dispenser 1 but is provided with a central duct 280' and two lateral ducts 282' and 284' which differ due to their respective geometry from the homologous ducts of the dispenser 1. In fact, in this case the central duct 280' opens towards the outside with a flared central nozzle 2800' having rectangular cross section, the geometrical shape of which is equal to the one described above with reference to the central nozzle 2800, to produce a fan-shaped jet which expands both in the direction of the first frontal median plane M and in the direction of the second sagittal median plane N in direct proportion to the widths of the nozzle 2800 measured along the directions identified by said planes. Below said central nozzle 2800', the central duct 280' has a rectangular section transversally to the axis A with decreasing shape and dimension from the bottom towards the dome 12. Also the lateral ducts 282' and 284' have a decreasing rectangular section towards the dome 12. At the base, on the side of the tapered portion 26, the lateral ducts 282' and 284' are arranged in a symmetrically polar manner relative to the axis A with reference to the sagittal plane N but end in a curved portion which is arranged laterally to said sagittal plane N on opposite quadrants, as illustrated in figure 12. The figures 14-16 facilitate understanding of the geometry of the ducts 280', 282' and 284' along the axis A. The central nozzle 2800' can be seen in section in figure 17, from which it is evident that the respective greater dimension develops symmetrically to the median plane M. Also in this case, the dome 12 has a faceted top 12' provided with the central flat portion 120' transversal to the axis A and four flat faces inclined with respect to the axis A, comprising two equal opposite first faces 122', symmetrical with respect to the median plane M, and two equal opposite second faces 124', symmetrical with respect to the sagittal plane N. The two lateral ducts 282' and 284' open at the top of the dome 12' with rectangular first and second lateral nozzles 2820' and 2840' with rounded vertexes and, further to the above description, inclined with respect to the axis A. What has been said with reference to the nozzles 2820 and 2840 also applies to the jets delivered from the first and second lateral nozzles 2820' and 2840'.

With reference to the enlargement of figure 17, the shape of the central nozzle 2800' is symmetrical with respect to the sagittal median plane N and has a first left-hand lateral portion 2801' which is spatially overlapping the first lateral nozzle 2820'. Naturally the same can be said for a second right-hand lateral portion 2802' and the second lateral nozzle 2840', due to the known rules of symmetry described above which eliminate the need for an additional figure without compromising understanding of the particular shape of the dispenser 1' overall. Therefore, also in this case the first and the second nozzles 2820' and 2840' are shaped to direct the respective lateral jets against the lateral portions of the fan-shaped jet coming out of the central nozzle 2800', determining, also in this case, a single jet of substantially nebulized particles, where said jet is shaped similar to the jet previously indicated as "modified fan-shaped jet".

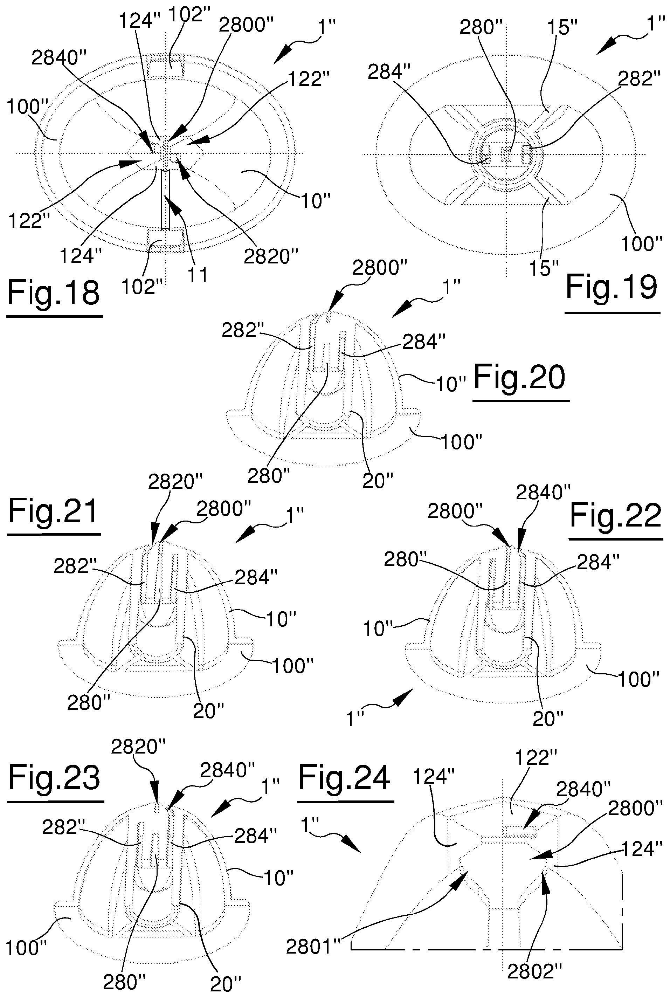

Furthermore, with reference to figures 18-24 a further variation of the dispenser 1 indicated by the reference number 1" is illustrated, where homologous structural details will be indicated for the sake of practicality by the same reference numbers as those used in figures 1-11, but provided with two apexes. In particular, the dispenser 1" is functionally and aesthetically similar to the dispenser 1 but is provided with a central duct 280'' and two lateral ducts 282" and 284" which differ in their respective geometry from the homologous ducts of the dispenser 1. In particular, with reference to figures 18-24, the central duct 280'' has a rectangular cross section with area decreasing in a linear manner from the base of the respective hollow lower portion 22" to the top of the dome 12, where it ends in a central nozzle 2800'' flared with section transversal to the axis A of rectangular shape, therefore delimited at the top by a rectangular edge having substantially constant width, measured according to the direction of the sagittal plane N. In particular, the central nozzle 2800" has a section transversal to the axis A which is again rectangular and has a constant width, but is profiled in longitudinal section (figure 24) according to a trend that starts linearly from the central duct 280" and ends in a curved concave manner at the top 12". Also in this case the first and the second nozzles 2820" and 2840" are arranged and shaped to direct the respective lateral jets against the lateral portions of the jet coming out of the rectangular flared central nozzle 2800'', determining a single jet shaped like the above-mentioned "modified fan-shaped jet". In this case it should be noted that the width of the upper end portion of the central nozzle 2800" is substantially double the extension of the first and second lateral nozzles 2820" and 2840'', and the particular arrangement of the latter as shown by figure 24 totally overlaps one half of the central nozzle 2800'', allowing the area of impact of the respective jets to be maximized, and therefore the width of the "modified fan-shaped jet" resulting from the impact.

The breakdown of the fan-shaped jet emitted from the central nozzles (2800, 2800' and 2800" respectively), due to the collision of the jets delivered by the first and second lateral nozzles (2820 and 2840, 2820' and 2840', 2820" and 2840" respectively) on the respective lateral portions of the fan-shaped jet, determines a fragmentation of the latter with modification and deviation, in all directions, of the particles that compose both the central fan-shaped jet and the lateral jets. Therefore, the particles of the jet produced by said fragmentation are uniformly distributed inside the entire nasal cavity, also reaching those areas of the nasal mucosa which it was not possible to reach with the devices currently on the market and assuming a substantially nebulized form, with very delicate impact on the structures composing the mucosa covering the nasal cavities and in particular on the mucociliary system.

Furthermore, the pressure with which the washing liquid is delivered through any one of the dispensers described above can be manually controlled if it is the result of pressure exerted manually on a bottle delimited by flexible walls or by a bag (known and not illustrated for the sake of economy of drawing) that contain the liquid and are connected to the dispenser 1 through the lower portion 22 of the second body 20, and mechanically controlled if the action of the user is exerted on a control member known and not illustrated which determines the pressure variation in said bottle.

Any excess pressure is modulated through the groove 11, which allows discharge of the excess solution.

Further to the above description, it can be seen that a dispenser shaped as described above and therefore provided with a central nozzle elongated according to a given plane able to deliver a fan-shaped jet according to said plane with polar symmetry with respect to the central nozzle and two lateral nozzles arranged on opposite sides of said plane and facing lateral portions of the central nozzle allows, in use (or when all these nozzles are supplied with a liquid under pressure), a single jet in a "modified fan" shape to be produced, the width of which is a function of the width of the central nozzle and the degree of overlapping between the lateral portions of said central nozzle and the lateral nozzles. The arrangement of the lateral nozzles, polarly symmetrical with respect to the axis of the central nozzle, gives a sort of rotation to the jet delivered by the central nozzle, which maximizes deviation of the liquid particles delivered by the central nozzle producing the "modified fan-shaped jet" where the spatial distribution of the liquid particles above the top of the dispenser is maximized and, therefore, allows spraying of the mucosa of the cavity to be treated and, in the case of nasal cavities, the mucosa covering them throughout their extension.

## Claims

1. A liquid dispenser (1) for medical uses wherein a first membrane body (10) is shaped symmetrically with respect to a first front median plane (M) and to a second sagittal median plane (N) intersected in a central axis (A) and is delimited at the top by a dome (12); an extension of said first body (10) according to said second sagittal median plane (N) being greater than an extension of said first body (10) according to said first front median plane (M); a second body (20) being carried by said first body (10) inside said dome (12) in a concentric position to said axis (A); said second body (20) having a lower hollow portion (22) concentric with said axis (A) and an upper portion (28) that has a central duct (280) and at least a first lateral duct (282)(284)(282')(284')(282")(284") ending in said dome (12), respectively, with a central nozzle (2800)(2800')(2800") and at least a first lateral nozzle (2820)(2840)(2820')(2840')(2820")(2840"); wherein the projection of said central nozzle (2800)(2800')(2800'') on a plane perpendicular to said axis (A) has an oval or rectangular shape, said central nozzle (2800)(2800')(2800'') has a first lateral portion (2801)(2802)(2801')(2802')(2801")(2802'') the projection of which on said plane perpendicular to said axis (A) extends laterally with respect to said second sagittal median plane (N), and the projection on said plane perpendicular to said axis (A) of said at least one first lateral nozzle (2820)(2840)(2820')(2840')(2820")(2840") is positioned entirely laterally with respect to said second sagittal median plane (N).

2. The dispenser (1) according to claim 1, **characterized in that** the projection on said first frontal median plane (M) of said at least one first lateral duct (282) (284) (282') (284') (282'') (284'') is at least partially overlapping the projection on said first frontal median plane (M) of said central duct (280).

3. The dispenser (1) according to claim 1 or 2, **characterized in that** the projection on said plane perpendicular to the axis (A) of said at least one first lateral nozzle (2820) (2840)(2820') (2840') (2820")(2840") has an oval or rectangular shape.

4. The dispenser according to one of the claims from 1 to 3, **characterized in that** said central nozzle (2800'') has a second lateral portion (2801'')(2802''), the projection of which on said plane perpendicular to said axis (A) extends laterally with respect to said second sagittal median plane (N) and symmetrically with respect to said projection on said plane perpendicular to said axis (A) of said first portion of central nozzle.

5. The dispenser according to claim 4, **characterized in that** said dispenser (1) comprises a second lateral duct ending in said dome (12) with a second lateral nozzle, the projection of which on said plane perpendicular to said axis (A) is positioned entirely laterally with respect to said second sagittal median plane (N).

6. The dispenser (1) according to claim 4 or 5, **characterized in that** the projection on said first frontal median plane (M) of said at least one second lateral duct (282) (284) (282') (284') (282'') (284'') at least partially overlaps the projection on said first frontal median plane (M) of said central duct (280).

7. The dispenser (1) according to claim 5 or 6, **characterized in that** the projection on said plane perpendicular to the axis (A) of said at least one second lateral nozzle (2820) (2840) (2820') (2840') (2820")(2840") has an oval or rectangular shape.

8. The dispenser according to any one of the preceding claims, **characterized in that** said central nozzle (2800)(2800')(2800") is flared and has a section increasing towards said dome (12).

9. The dispenser according to any one of the preceding claims from 5 to 8, **characterized in that** said first and second lateral ducts (282) (284) are developed on parallel planes.

10. The dispenser according to any one of the preceding claims, **characterized in that** said dome (12) has externally at least one discharge groove (11).

## Patentansprüche

1. Ein Flüssigkeitsspender (1) für die medizinische Anwendung, wobei ein erster Membrankörper (10) symmetrisch in Bezug auf eine erste frontale mediane Ebene (M) und eine zweite sagittale mediane Ebene (N), die sich in einer zentralen Achse (A) schneiden, geformt ist und an der Oberseite durch eine Kuppel (12) begrenzt ist; wobei eine Ausdehnung des ersten Körpers (10) in Bezug auf die zweite sagittale mediane Ebene (N) größer als eine Ausdehnung des ersten Körpers (10) in Bezug auf die erste vordere mediane Ebene (M) ist;
wobei von dem ersten Körper (10) innerhalb der Kuppel (12) ein zweiter Körper (20) in einer konzentrischen Position hinsichtlich der Achse (A) getragen wird; wobei der zweite Körper (20) einen unteren hohlen Abschnitt (22), der konzentrisch hinsichtlich der Achse (A) ist, und einen oberen Abschnitt (28) mit einem zentralen Kanal (280) und mindestens einem ersten lateralen Kanal (282) (284) (282') (284') (282") (284"), der in der Kuppel (12) endet, jeweils mit einer zentralen Düse (2800) (2800') (2800") und mindestens einer ersten lateralen Düse (2820) (2840) (2820') (2840') (2820") (2840"), aufweist; wobei
die Projektion der zentralen Düse (2800) (2800') (2800") auf eine Ebene senkrecht zu der Achse (A) eine ovale oder rechteckige Form aufweist, die zentrale Düse (2800) (2800') (2800") einen ersten lateralen Abschnitt (2801) (2802) (2801') (2802') (2801") (2802") aufweist, dessen Projektion auf die Ebene senkrecht zu der Achse (A) sich lateral in Bezug auf die zweite sagittale mediane Ebene (N) ausdehnt, und die Projektion der mindestens einen ersten lateralen Düse (2820) (2840) (2820') (2840') (2820") (2840") auf die Ebene senkrecht zu der Achse (A) vollständig lateral in Bezug auf die zweite sagittale mediane Ebene (N) positioniert ist.

2. Spender (1) gemäß Anspruch 1, **dadurch gekennzeichnet, dass** die Projektion auf die erste frontale mediane Ebene (M) des mindestens einen ersten lateralen Kanals (282) (284) (282') (284') (282") (284") mindestens zum Teil die Projektion auf die erste frontale mediane Ebene (M) des zentralen Kanals (280) überlappt.

3. Spender (1) gemäß Anspruch 1 oder 2, **dadurch gekennzeichnet, dass** die Projektion auf die Ebene senkrecht zu der Achse (A) der mindestens einen ersten lateralen Düse (2820) (2840) (2820') (2840') (2820") (2840") eine ovale oder rechteckige Form aufweist.

4. Spender gemäß einem der Ansprüche 1 bis 3, **dadurch gekennzeichnet, dass** die zentrale Düse (2800") einen zweiten lateralen Abschnitt (2801") (2802") aufweist, dessen Projektion auf die Ebene senkrecht zu der Achse (A) sich lateral in Bezug auf die zweite sagittale mediane Ebene (N) und symmetrisch in Bezug auf die Projektion auf die Ebene senkrecht zu der Achse (A) des ersten Abschnitts der zentralen Düse ausdehnt.

5. Spender gemäß Anspruch 4, **dadurch gekennzeichnet, dass** der Spender (1) einen zweiten lateralen Kanal beinhaltet, der in der Kuppel (12) mit einer zweiten lateralen Düse endet, deren Projektion auf die Ebene senkrecht zu der Achse (A) vollständig lateral in Bezug auf die zweite sagittale mediane Ebene (N) positioniert ist.

6. Spender (1) gemäß Anspruch 4 oder 5, **dadurch gekennzeichnet, dass** die Projektion auf die erste frontale mediane Ebene (M) des mindestens einen zweiten lateralen Kanals (282) (284) (282') (284') (282") (284") mindestens zum Teil mit der Projektion auf die erste frontale mediane Ebene (M) des zentralen Kanals (280) überlappt.

7. Spender (1) gemäß Anspruch 5 oder 6, **dadurch gekennzeichnet, dass** die Projektion auf die Ebene senkrecht zu der Achse (A) der mindestens einen zweiten lateralen Düse (2820) (2840) (2820') (2840') (2820") (2840") eine ovale oder rechteckige Form aufweist.

8. Spender gemäß einem der vorhergehenden Ansprüche, **dadurch gekennzeichnet, dass** die zentrale Düse (2800) (2800') (2800") aufgeweitet ist und einen in Richtung der Kuppel (12) zunehmenden Querschnitt aufweist.

9. Spender gemäß einem der vorhergehenden Ansprüche 5 bis 8, **dadurch gekennzeichnet, dass** der erste und der zweite laterale Kanal (282) (284) auf parallelen Ebenen angeordnet sind.

10. Spender gemäß einem der vorhergehenden Ansprüche, **dadurch gekennzeichnet, dass** die Kuppel (12) extern mindestens eine Abflussrille (11) aufweist.

## Revendications

1. Un distributeur de liquide (1) destiné à des utilisations médicales où un premier corps de membrane (10) est formé de manière symétrique par rapport à un premier plan médian frontal (M) et à un deuxième plan médian sagittal (N) se croisant dans un axe central (A) et est délimité au niveau du dessus par un dôme (12) ; une étendue dudit premier corps (10) relativement audit deuxième plan médian sagittal (N) étant supérieure à une étendue dudit premier corps (10) relativement audit premier plan médian frontal (M) ;
un deuxième corps (20) étant porté par ledit premier corps (10) à l'intérieur dudit dôme (12) dans une position concentrique relativement audit axe (A) ; ledit deuxième corps (20) présentant une portion creuse inférieure (22) concentrique relativement audit axe (A) et une portion supérieure (28) qui présente un conduit central (280) et au moins un premier conduit latéral (282) (284) (282') (284') (282") (284") se terminant dans ledit dôme (12), respectivement, avec une buse centrale (2800) (2800') (2800") et au moins une première buse latérale (2820) (2840) (2820') (2840') (2820") (2840") ; où
la projection de ladite buse centrale (2800) (2800') (2800") sur un plan perpendiculaire audit axe (A) présente une forme ovale ou rectangulaire, ladite buse centrale (2800) (2800') (2800") présentant une première portion latérale (2801) (2802) (2801') (2802') (2801") (2802") dont la projection sur ledit plan perpendiculaire audit axe (A) s'étend latéralement par rapport audit deuxième plan médian sagittal (N), et la projection sur ledit plan perpendiculaire audit axe (A) de ladite au moins une première buse latérale (2820) (2840) (2820') (2840') (2820") (2840") est positionnée entièrement latéralement par rapport audit deuxième plan médian sagittal (N).

2. Le distributeur (1) selon la revendication 1, **caractérisé en ce que** la projection sur ledit premier plan médian frontal (M) dudit au moins un premier conduit latéral (282) (284) (282') (284') (282") (284") chevauche au moins partiellement la projection sur ledit premier plan médian frontal (M) dudit conduit central (280).

3. Le distributeur (1) selon la revendication 1 ou la revendication 2, **caractérisé en ce que** la projection sur ledit plan perpendiculaire à l'axe (A) de ladite au moins une première buse latérale (2820) (2840) (2820') (2840') (2820") (2840") présente une forme ovale ou rectangulaire.

4. Le distributeur selon l'une des revendications allant de 1 à 3, **caractérisé en ce que** ladite buse centrale (2800") présente une deuxième portion latérale (2801") (2802"), dont la projection sur ledit plan perpendiculaire audit axe (A) s'étend latéralement par rapport audit deuxième plan médian sagittal (N) et de manière symétrique par rapport à ladite projection sur ledit plan perpendiculaire audit axe (A) de ladite première portion de buse centrale.

5. Le distributeur selon la revendication 4, **caractérisé en ce que** ledit distributeur (1) comprend un deuxième conduit latéral se terminant dans ledit dôme (12) avec une deuxième buse latérale, dont la projection sur ledit plan perpendiculaire audit axe (A) est positionnée entièrement latéralement par rapport audit deuxième plan médian sagittal (N).

6. Le distributeur (1) selon la revendication 4 ou la revendication 5, **caractérisé en ce que** la projection sur ledit premier plan médian frontal (M) dudit au moins un deuxième conduit latéral (282) (284) (282') (284') (282") (284") chevauche au moins partiellement la projection sur ledit premier plan médian frontal (M) dudit conduit central (280).

7. Le distributeur (1) selon la revendication 5 ou la revendication 6, **caractérisé en ce que** la projection sur ledit plan perpendiculaire à l'axe (A) de ladite au moins une deuxième buse latérale (2820) (2840) (2820') (2840') (2820") (2840") présente une forme ovale ou rectangulaire.

8. Le distributeur selon l'une quelconque des revendications précédentes, **caractérisé en ce que** ladite buse centrale (2800) (2800') (2800") est évasée et présente une section s'accroissant vers ledit dôme (12).

9. Le distributeur selon l'une quelconque des revendications précédentes allant de 5 à 8, **caractérisé en ce que** lesdits premier et deuxième conduits latéraux (282) (284) sont développés sur des plans parallèles.

10. Le distributeur selon l'une quelconque des revendications précédentes, **caractérisé en ce que** ledit dôme (12) présente sur l'extérieur au moins une rainure d'évacuation (11).
